# EUROPEAN PATENT SPECIFICATION

(11) **EP 3 513 734 B1**
(45) Date of publication and mention of the grant of the patent: **13.09.2023**
(21) Application number: 18206964.1
(22) Date of filing: 19.11.2018
(51) Int. Cl.: A61B 8/00

(54) **ULTRASONIC IMAGING APPARATUS AND CONTROL METHOD THEREOF**
ULTRASCHALLABBILDUNGSVORRICHTUNG UND STEUERUNGSVERFAHREN DAFÜR
APPAREIL D'IMAGERIE ULTRASONIQUE ET SON PROCÉDÉ DE CONTRÔLE

(30) Priority: 16.01.2018 KR 20180005330
(43) Date of publication of application: 24.07.2019
(73) Proprietor: Samsung Medison Co., Ltd., Hongcheon-gun, Gangwon-do, 25108 (KR)
(72) Inventor: SONG, In Seong, Daegu (KR); LEE, Jong Mok, Gyeonggi-do (KR); OH, Won Gee, Gyeonggi-do (KR)
(74) Representative: Hylarides, Paul Jacques

(56) References cited:
- WO-A1-2013/100245
- JP-A- 2007 037 587
- US-A1- 2003 011 285
- US-A1- 2011 040 187
- US-A1- 2013 338 503
- US-B2- 7 438 684

## Description

### BACKGROUND

### 1. Field of the Invention

The present disclosure relates to an ultrasonic imaging apparatus and control method thereof, and more particularly, to an ultrasonic imaging apparatus and control method thereof, which improves the resolution of ultrasound images by compensating for deviations or variations of thickness of a membrane cap and 3D oil of an ultrasonic probe.

### 2. Discussion of Related Art

Ultrasonic imaging apparatuses irradiate ultrasound signals from the surface of a body of a subject toward a particular region inside the body, and noninvasively obtain a cross-sectional tomogram of a soft tissue or an image of the blood flow using a reflected ultrasound signal (or echo ultrasound signal).

Compared to other diagnostic imaging apparatuses, such as X-ray diagnostic apparatuses, X-ray Computerized Tomography (CT) scanners, Magnetic Resonance Imaging (MRI) apparatuses, nuclear medicine diagnostic apparatuses, etc., ultrasonic imaging apparatuses have many advantages that they are compact, inexpensive, able to display in real time, and safe because of no exposure to radiation, and are thus widely used for diagnosis in cardiology clinics, abdominal pain clinics, urology clinics , and women clinics.

The ultrasonic imaging apparatus typically has an ultrasonic probe to transmit ultrasound to an object and receive echo ultrasound from the object while directly contacting the object. The ultrasonic probe is equipped with a cap to cover transducers or an acoustic coupler to efficiently transmit ultrasound energy to the object at the front end, and in the conventional technologies, distortion of ultrasound images caused by the thickness of the cap or acoustic coupler is not considered.

Document US7438684 discloses a ultrasonic diagnostic apparatus monitoring the thickness of a cap and an acoustic coupler attached to an ultrasound probe in order to estimate the surface temperature of the probe. Document US2003/011285 discloses calculation and application of beam-former delays to compensate for the shape of an ultrasound probe protective cover.

In the conventional technologies, it is hard to obtain accurate ultrasound images because variations of the form of the cap and acoustic coupler, which occur due to the pressure applied while the ultrasonic probe contacts and scans the subject, is not taken into account.

Moreover, in the conventional technologies, operation for processing ultrasound signals is performed on the assumption that thickness of the cap is uniform without considering that actual caps are likely to have irregular thickness in the manufacturing process of the cap, and accordingly, there might be distortions in the ultrasound image.

### SUMMARY OF THE INVENTION

The invention is defined in the independent claims.

The present disclosure provides an ultrasonic imaging apparatus and control method thereof, capable of compensating for distortions of ultrasonic transducer signals by measuring thickness of a membrane cap and three dimensional (3D) oil covering the ultrasonic transducer in real time without additional hardware.

The present disclosure also provides an ultrasonic imaging apparatus and control method thereof, capable of improving the resolution of ultrasound images by compensating for thickness deviations of positions on a membrane cap and performing a compensation process on curvature changes of the membrane cap and thickness changes of 3D oil.

The present disclosure also provides an ultrasonic imaging apparatus and control method thereof, capable of securing uniformity of signal sensitivity of ultrasonic transducer elements by performing compensation for irregularity in thickness of a membrane cap or 3D oil.

According to an embodiment of the present disclosure, an ultrasonic imaging apparatus may comprises an ultrasonic probe configured to have an ultrasonic transducer with a plurality of elements, a membrane cap provided in a form that covers the ultrasonic transducer, and three dimensional (3D) oil provided between the ultrasonic transducer and the membrane cap; a controller configured to obtain thickness data of at least one of the membrane cap and the 3D oil to compensate for a distortion of a signal transmitted or received by the ultrasonic transducer; and a display configured to display an ultrasonic image created based on a ultrasonic transducer signal compensated by the controller.

The controller may be configured to obtain thickness data of the 3D oil based on time taken for an ultrasound signal from the ultrasonic transducer to reflect off on a border between the membrane cap and the 3D oil and reach a surface of the ultrasonic transducer and ultrasound velocity in the 3D oil.

The controller may be configured to obtain thickness data of the membrane cap based on time for an ultrasound signal from the ultrasonic transducer to reflect off on an outer surface of the membrane cap and reach a border between the membrane cap and the 3D oil from when the ultrasound signal penetrates the border between the membrane cap and the 3D oil and ultrasound velocity in the membrane cap.

The controller is configured to calculate time delay values to be applied to signals transmitted or received by the respective ultrasonic transducer elements by reflecting thickness deviations of the membrane cap or the 3D oil.

The controller may be configured to obtain thickness data of the membrane cap for the entire area of the membrane cap in the early stage of operation of the ultrasonic imaging apparatus, and compensate for thickness deviations of positions on the membrane cap based on the thickness data of the membrane cap corresponding to a wavelength of the ultrasonic transducer signal.

The controller may be configured to obtain thickness data of the membrane cap or the 3D oil in real time to compensate for a distortion of a signal transmitted or received by the ultrasonic transducer.

The controller may be configured to determine the magnitude of an echo signal reflecting off on a border between the membrane cap and the 3D oil for each of the plurality of ultrasonic transducer elements, regulate a pulse voltage to be applied to each of the ultrasonic transducer elements to make the magnitude of the ultrasound signals transmitted by the plurality of ultrasonic transducer elements uniform.

The controller may be configured to regulate pulse voltages to be applied to the respective ultrasonic transducer elements to compensate for differences in magnitude between ultrasound signals transmitted and echo signals received by the respective ultrasonic transducer elements. The membrane cap may be formed of plastics or rubber.

According to another embodiment of the present disclosure, a control method of an ultrasonic imaging apparatus including an ultrasonic probe configured to have an ultrasonic transducer with a plurality of elements, a membrane cap provided in a form that covers the ultrasonic transducer, and three dimensional (3D) oil provided between the ultrasonic transducer and the membrane cap, the control method may comprise obtaining thickness data of at least one of the membrane cap and the 3D oil; compensating for a distortion of a signal transmitted or received by the ultrasonic transducer based on the thickness data; and displaying an ultrasonic image created based on the compensated ultrasonic transducer signal.

The obtaining of the thickness data may comprise obtaining thickness data of the 3D oil based on time taken for an ultrasound signal from the ultrasonic transducer to reflect off on a border between the membrane cap and the 3D oil and reach a surface of the ultrasonic transducer and ultrasound velocity in the 3D oil.

The obtaining of the thickness data may comprise obtaining thickness data of the membrane cap based on time for an ultrasound signal from the ultrasonic transducer to reflect off on an outer surface of the membrane cap and reach a border between the membrane cap and the 3D oil from when the ultrasound signal penetrates the border between the membrane cap and the 3D oil and ultrasound velocity in the membrane cap.

The compensating for the distortion of the signal comprises calculating time delay values to be applied to signals transmitted or received by the respective ultrasonic transducer elements by reflecting thickness deviations of the membrane cap or the 3D oil.

The obtaining of the thickness data may comprise obtaining thickness data of the membrane cap for the entire area of the membrane cap in the early stage of operation of the ultrasonic imaging apparatus, and wherein the compensating for the distortion of the signal comprises compensating for thickness deviations of positions on the membrane cap based on the thickness data of the membrane cap corresponding to a wavelength of the ultrasonic transducer signal.

The obtaining of the thickness data may comprise obtaining thickness data of the membrane cap or the 3D oil in real time.
the control method may further comprise determining the magnitude of an echo signal reflecting off on a border between the membrane cap and the 3D oil for each of the plurality of ultrasonic transducer elements, and regulating a pulse voltage to be applied to each of the ultrasonic transducer elements to make the magnitude of the ultrasound signals transmitted by the plurality of ultrasonic transducer elements uniform.

The regulating of the pulse voltage may comprise regulating pulse voltages to be applied to the respective ultrasonic transducer elements to compensate for differences in magnitude between ultrasound signals transmitted and echo signals received by the respective ultrasonic transducer elements.

The membrane cap may be formed of plastics or rubber.

According to an embodiment of the present disclosure, an ultrasonic imaging apparatus and control method thereof is capable of compensating for distortions of ultrasonic transducer signals by measuring thickness of a membrane cap and 3D oil covering the ultrasonic transducer in real time without additional hardware, thereby saving manufacturing costs.

According to an embodiment of the present disclosure, an ultrasonic imaging apparatus and control method thereof is capable of improving the resolution of ultrasound images by compensating for thickness deviations of positions on a membrane cap and performing a compensation process on curvature changes of the membrane cap and thickness changes of 3D oil.

According to an embodiment of the present disclosure, an ultrasonic imaging apparatus and control method thereof is capable of securing uniformity of signal sensitivity of ultrasonic transducer elements by performing compensation for irregularity in thickness of a membrane cap or 3D oil.

### BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
FIG. 1 shows the exterior of an ultrasonic imaging apparatus, according to an embodiment of the present disclosure;
FIG. 2 shows internal features of the front end of an ultrasonic probe, according to an embodiment of the present disclosure;
FIG. 3A, 3B, 3C shows views for explaining thickness deviations of a membrane cap and three dimensional (3D) oil, according to an embodiment of the present disclosure;
FIG. 4 is a control block diagram of an ultrasonic imaging apparatus, according to an embodiment of the present disclosure;
FIG. 5 is a detailed control block diagram of a main body of an ultrasonic imaging apparatus, according to an embodiment of the present disclosure;
FIG. 6 shows detailed control block diagrams of a transmitter and a receiver, according to an embodiment of the present disclosure;
FIG. 7 is a view for explaining beamforming of an ultrasonic transducer element signal, according to an embodiment of the present disclosure;
FIG. 8 is a view for explaining a method for obtaining thickness data of a membrane cap and 3D oil, according to an embodiment of the present disclosure; and
FIG. 9 is a flowchart illustrating a control method of an ultrasonic imaging apparatus, according to an embodiment of the present disclosure.

### DETAILED DESCRIPTION OF EXEMPLARY EMBODIMENTS

Reference will now be made in detail to embodiments, examples of which are illustrated in the accompanying drawings, wherein like reference numerals refer to the like elements throughout. The terms "front", "rear", "upper", "lower", "top", and "bottom" as herein used are defined with respect to the drawings, but the terms may not restrict the shape and position of the respective components.

Like numerals refer to like elements throughout the specification. Not all elements of embodiments of the present disclosure will be described, and description of what are commonly known in the art or what overlap each other in the embodiments will be omitted. The terms as used throughout the specification, such as "~ part", "~ module", "~ member", "~ block", etc., may be implemented in software and/or hardware, and a plurality of "- parts", "- modules", "- members", or "- blocks" may be implemented in a single element, or a single "- part", "- module", "~ member", or "- block" may include a plurality of elements.

It will be further understood that the term "connect" or its derivatives refer both to direct and indirect connection, and the indirect connection includes a connection over a wireless communication network.

The term "include (or including)" or "comprise (or comprising)" is inclusive or open-ended and does not exclude additional, unrecited elements or method steps, unless otherwise mentioned.

Throughout the specification, when it is said that a member is located "on" another member, it implies not only that the member is located adjacent to the other member but also that a third member exists between the two members.

It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section.

It is to be understood that the singular forms "a," "an," and "the" include plural references unless the context clearly dictates otherwise.

Reference numerals used for method steps are just used for convenience of explanation, but not to limit an order of the steps. Thus, unless the context clearly dictates otherwise, the written order may be practiced otherwise.

The principle and embodiments of the present invention will now be described with reference to accompanying drawings.

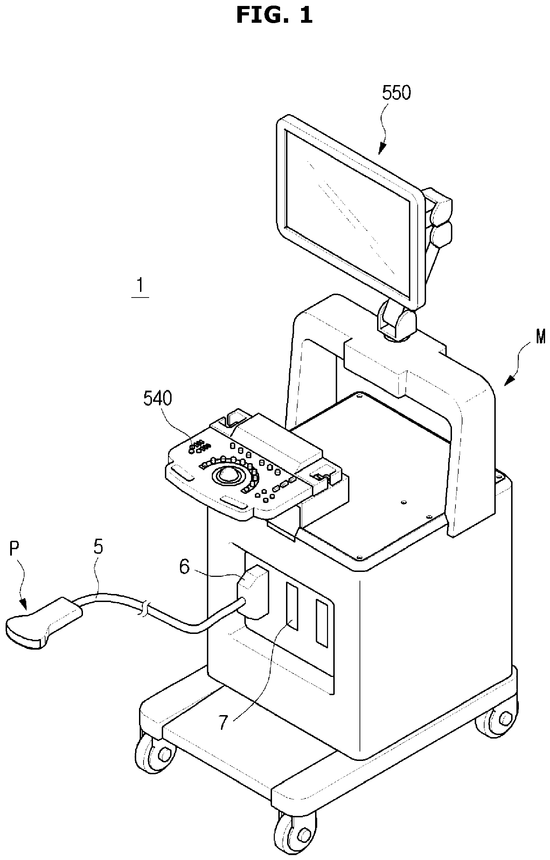

FIG. 1 shows the exterior of an ultrasonic imaging apparatus, according to an embodiment of the present disclosure.

Referring to FIG. 1, an ultrasonic imaging apparatus 1 may include an ultrasonic probe P that transmits ultrasounds to an object, receives ultrasound signals from the object and converts the echo ultrasound signals to an electric signal, and a main body M connected to the ultrasonic probe P and equipped with an input 540 and a display 550 for displaying ultrasound images. The ultrasonic probe P may be connected to the main body M of the ultrasonic imaging apparatus 1 via a cable 5, for receiving various signals required to control the ultrasonic probe P or forwarding analog or digital signals that correspond to echo ultrasound signals received by the ultrasonic probe P to the main body M. However, embodiments of the ultrasonic probe P are not limited thereto, and may be implemented with a wireless probe to exchange signals with the main body M over a network formed between the ultrasonic probe P and the main body M.

One end of the cable 5 may be connected to the ultrasonic probe P, and the other end of the cable 5 may be connected to a connector 6 that may be coupled to or decoupled from a slot 7 of the main body M. The main body M and the ultrasonic probe P may exchange control commands or data via the cable 5. For example, when the user inputs information about a focal depth, an aperture size or shape, a steering angle or the like through the input 540, the information may be sent to the ultrasonic probe P via the cable 5 and used for transmit and receive beamforming of a transmitter 100 and a receiver 200, respectively. Alternatively, in the case that the ultrasonic probe P is implemented with a wireless probe as mentioned above, the ultrasonic probe P is connected to the main body M not through the cable 5 but through a wireless network. Even in the case that the ultrasonic probe P is connected to the main body M over a wireless network, control commands or data may be exchanged between the main body M and the ultrasonic probe P.

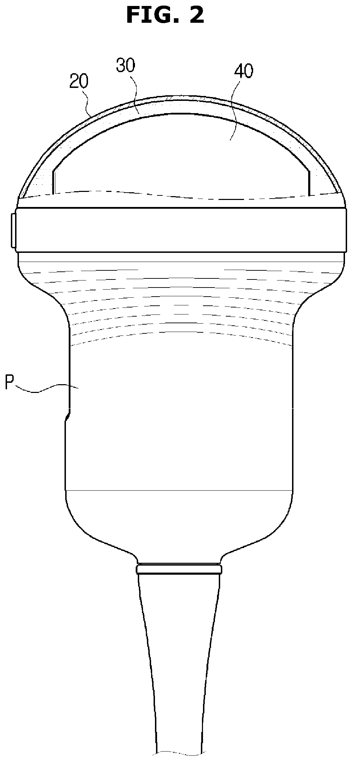

FIG. 2 shows internal features of the front end of an ultrasonic probe, according to an embodiment of the present disclosure.

Referring to FIG. 2, the front end of the ultrasonic probe P includes a membrane cap 20, three dimensional (3D) oil 30, and an ultrasonic transducer 40. The membrane cap 20 is provided in the form that covers the ultrasonic transducer 40, and the 3D oil 30 is provided between the membrane cap 20 and the ultrasonic transducer 40. The membrane cap 20 may be of a convex type with a convexly curved middle portion, or of a linear type with a flat plane. It is not, however, limited thereto.

The membrane cap 20 may be formed of plastics or rubber. That is, the membrane cap 20 may be formed of an elastic material with low ultrasound transmission loss and high heat conductivity. If the membrane cap 20 is formed of a soft elastic material, the membrane cap 20 and the 3D oil 30 may be deformed by the pressure applied to scan the object. On the other hand, if the membrane cap 20 is formed of a rigid material, the membrane cap 20 may not be significantly deformed even when pressure is applied thereto while the ultrasonic probe P contacts and pushes the object.

The 3D oil 30 is a transmission medium for efficiently transmitting ultrasound energy to the object, and may have as low attenuation and acoustic impedance as the tissue of the object. The 3D oil 30 is a fluid, which may be fluidly deformed by the pressure applied to the membrane cap 20 when the object is scanned.

The ultrasonic transducer 40 includes an array of a plurality of elements 50. The ultrasonic transducer 40 may generate ultrasound while vibrating by an input pulse signal or alternate current and receive echo ultrasound reflecting and returning from a target portion of the object. Furthermore, the ultrasonic transducer 40 may be rotated to a certain angle within the membrane cap 20.

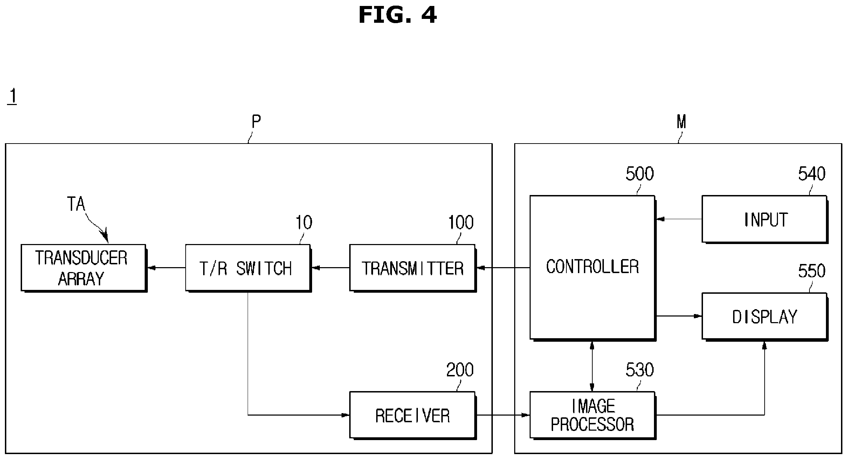

Operation of the ultrasonic transducer 40 will be described later in connection with FIGS. 4 to 7.

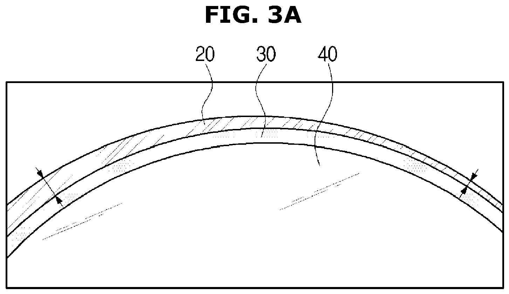

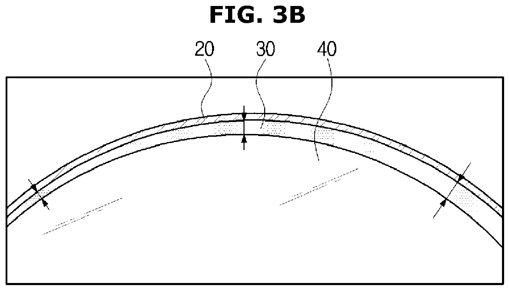

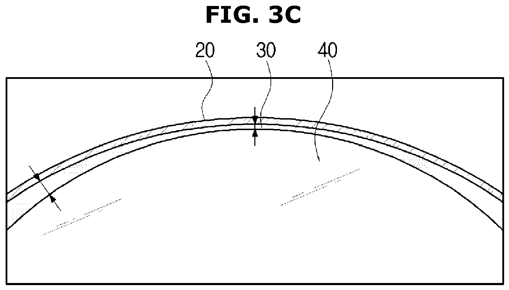

FIG. 3A, 3B, 3C shows views for explaining thickness deviations of a membrane cap and 3D oil, according to an embodiment of the present disclosure.

Referring to FIG. 3, the membrane cap 20 has a hemispherical form that seals the ultrasonic transducer 40, and is manufactured to have minimum thickness to reduce attenuation or distortion of signals to be transmitted or received by the ultrasonic transducer 40. By the nature of the manufacturing process, the membrane cap 20 may be manufactured to be typically in thickness of about 0.8 mm with an error of about 0.08 mm. However, in the process of manufacturing the membrane cap 20, it is very difficult to form the entire area of the membrane cap 20 to have uniform thickness as thin as possible. There is a difference in thickness between a designed membrane cap and an actually manufactured membrane cap.

Furthermore, there may be differences in thickness between positions on the membrane cap 20. In other words, the entire area of the membrane cap 20 may not have uniform thickness. FIG. 3A shows an example of thickness deviations of the actually manufactured membrane cap 20. In actual practice, the membrane cap 20 is formed such that a terminal portion of the membrane cap 20 coupled with a base frame of the ultrasonic probe is thicker than the middle portion of the membrane cap 20. Also, referring to FIG. 3B, the thickness of the 3D oil 30 may not be uniform when the positions of the membrane cap 20 and the ultrasonic transducer 40 are not accurately matched in the process of assembling or processing the membrane cap 20 and the 3D oil 30. FIG. 3C shows thickness deviations of the 3D oil 30 occurring when the ultrasonic probe P contacts and pushes the object.

As such, if there are thickness deviations in the membrane cap 20 or the 3D oil 30 in the manufacturing process, ultrasound energy to be transmitted to the object is different at every position on the membrane cap 20, and thus the ultrasound signals are not uniformly transmitted. Even the echo ultrasound signals are not uniformly received. This may lead to a problem that the resolution and quality of an ultrasound image deteriorate.

The present disclosure overcomes the problem by obtaining thickness data for the entire area of the membrane cap 20 and compensating for thickness deviations of positions on the membrane cap 20, thereby securing uniformity of signals transmitted or received by the ultrasonic transducer 40.

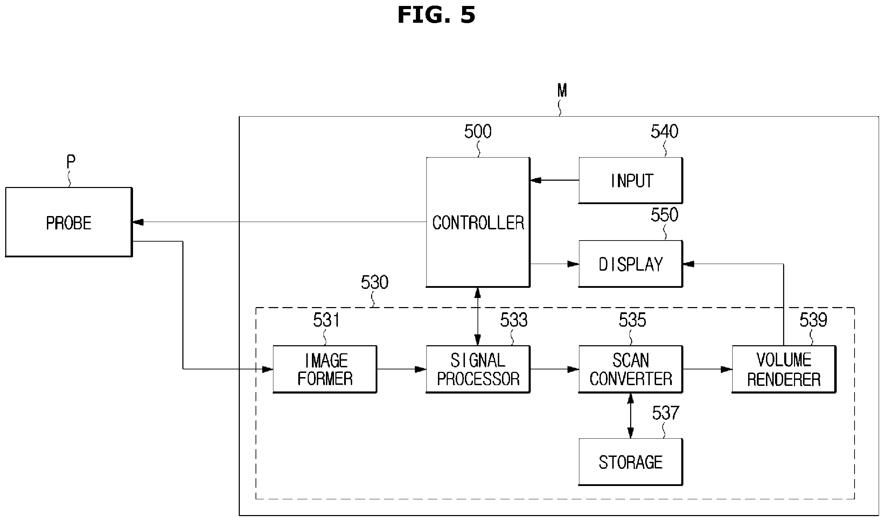

FIG. 4 is a control block diagram of an ultrasonic imaging apparatus, according to an embodiment of the present disclosure, and FIG. 5 is a detailed control block diagram of a main body of an ultrasonic imaging apparatus, according to an embodiment of the present disclosure.

Referring to FIG. 4, the main body M may include a controller 500, an image processor 530, an input 540, and a display 550.

The controller 500 controls general operation of the ultrasonic imaging apparatus 1. Specifically, the controller 500 generates control signals to control the respective components of the ultrasonic imaging apparatus 1, e.g., a Transmission/Reception (T/R) switch 10, the transmitter 100, the receiver 200, the image processor 530, the display 550, etc., as shown in FIG. 4.

Although in the ultrasonic imaging apparatus 1 according to an embodiment as shown in FIGS. 4 and 5, a transceiving beamformer may be included not in the main body M but in the ultrasonic probe P, the transceiving beamformer may be included not in the ultrasonic probe P but in the main body M in some other embodiments.

The controller 500 may calculate a delay profile for the plurality of ultrasonic transducer elements 50 that constitute an ultrasonic transducer array (TA), and calculate time delay values depending on differences in distance between the plurality of ultrasonic transducer elements 50 included in the ultrasonic transducer array (TA) and a focal point of the object based on the calculated delay profile. The controller 500 may then control the transceiving beamformer accordingly to generate transmit/receive signals.

The controller 500 may also generate control instructions for the respective components of the ultrasonic imaging apparatus 1 to control the ultrasonic imaging apparatus 1, according to instructions or commands input from the user through the input 540.

The image processor 530 may create an ultrasound image of a target portion inside the object based on the ultrasound signal focused by the receiver 200.

Referring to FIG. 4, the image processor 530 may include an image former 531, a signal processor 533, a scan converter 535, a storage 537, and a volume renderer 539.

The image former 531 may create a coherent two dimensional (2D) or 3D image of the target portion inside the object based on the ultrasound signal focused by the receiver 200.

The image processor 533 may convert the coherent image information formed by the image former 531 to ultrasonic image information for a diagnostic mode, such as Brightness mode (B-mode), Doppler mode (or D-mode), etc. For example, if the diagnostic mode is set to the B-mode, the signal processor 533 may perform e.g., an analog-to-digital (A/D) conversion process and compose ultrasonic image information in real time for a B-mode image. If a scan mode is set to the D-mode, the signal processor 533 may extract phase-change information from an ultrasound signal, calculate information about e.g., a blood flow at each point in the scanned cross-section, such as speed, power, or dispersion, and compose ultrasonic image information in real time for a D-mode image.

The scan converter 535 may convert the converted ultrasonic image information input from the signal processor 533 or the converted ultrasonic image information stored in the storage 537 to an ordinary video signal for display 550 and send the video signal to the volume renderer 539.

The storage 537 may temporarily or non-temporarily store the ultrasonic image information converted by the signal processor 533.

The volume renderer 539 may perform volume rendering based on the video signal sent from the scan converter 535, correct the rendered image information into a final resultant image, and send the resultant image to the display 550.

The input 540 may allow the user to input a command to operate the ultrasonic imaging apparatus 1. The user may input or set a command to start ultrasonic diagnosis, a command to select a diagnostic mode such as the B-mode, a motion mode (M-mode), the D-mode, an elasticity mode, a 3D mode, or the like, region-of-interest (ROI) setting information including size and location of the ROI.

The B mode is to display a cross-sectional image inside the object, representing portions having strong echoes and weak echoes in different brightness. A B-mode image is formed based on information acquired from tens to hundreds of scan lines.

The M-mode is to display an image representing how biological information (e.g., brightness information) of a particular portion (M line) in the cross-sectional image (B-mode image) of the object changes, and the B-mode image and the M-mode image are generally displayed on a single screen at the same time, allowing the user to compare and analyze two data to make a correct diagnosis.

The D-mode refers to a mode to display an image based on Doppler effect that causes a change in frequency of a sound output from a moving object. Such a mode using the Doppler effect may be further divided into Power Doppler Imaging (PDI) mode, color flow mode (S Flow), and Directional Power Doppler Imaging (DPDI) mode.

The PDI mode represents a level of a Doppler signal or the number of structures (e.g. the number of red blood cells in the blood) in an image that has no aliases because of being less sensitive to the incident angle, and has less image attenuation from noise. Furthermore, the PDI mode records reflected Doppler energy, and may thus be so sensitive that it is even able to detect narrow veins and slow blood streams.

The S Flow mode provides a power image representing power of a Doppler signal in a 2D distribution and a velocity image representing velocity of the Doppler signal. The image in the S Flow mode may not only visualize blood flow in real time but also represent a wide range of blood flow state from high-speed blood flow in a wide vein to low-speed blood flow in a narrow vein.

The DPDI mode refers to a mode to display a directional image that represents directional information of a Doppler signal in the PDI mode in a 2D distribution. Accordingly, the DPDI mode has an advantage over the PDI mode in that it allows more accurate detection of information regarding a blood flow. Furthermore, even for the Doppler mode image, an Mode image may be created.

The elasticity mode refers to a method for acquiring an elastographic image of the object using elastography. Elastography means analyzing a phenomenon that the harder the structure is like a malignant mass, the lower the elasticity of the tissues are and thus, the smaller the difference in metamorphosis of the tissues is. The elastographic image refers to an image representing stiffness of a tissue in quantity. Especially, it is used a lot in the field of diagnosing cervix, breast cancer or prostate cancer.

The 3D mode may refer to a mode to display an image indicating a geometric solid or space with x, y, z values representing depth, width, height, respectively, or to provide a 3D form to give a feeling of stereoscopy or display a series of images with the stereoscopic effect. For example, using the stereoscopic effect of the 3D mode, the user may display the shape of a face of a fetus and show it to its parents.

The input 540 may include various means for the user to input data, instructions, or commands, such as a keyboard, a mouse, a trackball, a tablet, a touch screen module, etc.

The display 550 may display menus or instructions required in ultrasonic diagnosis, and an ultrasound image obtained in the process of the ultrasonic diagnosis. The display 550 may display an ultrasound image of a target portion inside the object, which is created by the image processor 530. The ultrasound image to be displayed on the display 550 may be an ultrasound image in the B-mode or elastic mode, or may be a 3D ultrasound image. The display 550 may display various ultrasound images according to the aforementioned modes.

The display 550 may be implemented in various display methods known to the public, such as Cathode Ray Tube (CRT), Liquid Crystal Display (LCD), etc.

In an embodiment, as shown in FIG. 4, the ultrasonic probe P may include the transducer array TA, the T/R switch 10, the transmitter 100, and the receiver 200.

The transducer array TA is arranged at an end of the ultrasonic probe P. The ultrasonic transducer array TA refers to a 1D or 2D array of a plurality of ultrasonic transducer elements 50. The ultrasonic transducer array TA generates ultrasound while vibrating due to a pulse signal or alternate current (AC) applied thereto. The ultrasound is transmitted to a target portion inside the object. In this case, the ultrasound generated by the ultrasonic transducer array TA may be focused and transmitted to multiple target portions inside the object. That is, the ultrasound may be multifocused and transmitted to the multiple target portions.

The ultrasound generated by the ultrasonic transducer array TA may reflect off the target portion inside the object and may return to the ultrasonic transducer array TA. The ultrasonic transducer array TA may then receive the echo ultrasound reflecting and returning from the target portion. When the echo ultrasound arrives at the ultrasonic transducer array TA, the ultrasonic transducer array TA may oscillate at a certain frequency corresponding to a frequency of the echo ultrasound and output alternate current of a frequency corresponding to the oscillation frequency. Accordingly, the ultrasonic transducer array TA may convert the received echo ultrasound to a certain electric signal. Since each element 50 receives the echo ultrasound and outputs the electric signal, the ultrasonic transducer array TA may output electric signals on multiple channels.

The ultrasonic transducer 40 may be implemented by at least one of a magnetostrictive ultrasonic transducer that uses magnetostrictive effect of a magnetic substance, a piezoelectric ultrasonic transducer that uses piezoelectric effect of a piezoelectric substance, or a capacitive Micromachined Ultrasonic Transducer (cMUT) that transmits and receives ultrasound by means of oscillation of hundreds or thousands of micromachined thin films. In addition, other types of transducer that may generate ultrasound from an electrical signal or generate an electrical signal from ultrasound may also be an example of the aforementioned ultrasonic transducer.

For example, the ultrasonic transducer elements 50 may include piezoelectric oscillators or thin films. When alternate current is applied to the piezoelectric oscillators or thin films from a power source, the piezoelectric oscillators or thin films oscillate at a certain frequency due to the applied alternate current and generate ultrasound with the certain frequency. On the other hand, the piezoelectric oscillators or thin films oscillate due to an echo ultrasound with a certain frequency when the echo ultrasound reaches the piezoelectric oscillators or thin films, and output alternate current of a frequency corresponding to the oscillation frequency.

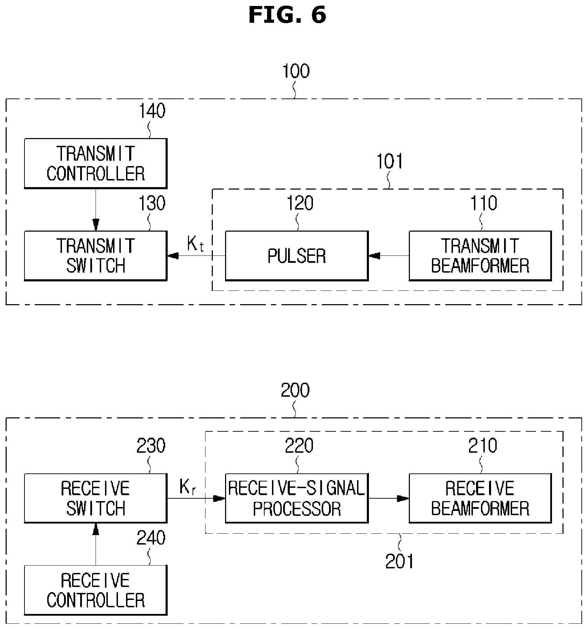

FIG. 6 is detailed control block diagrams of a transmitter and a receiver, according to an embodiment of the present disclosure.

Referring to FIG. 6, the transmitter 100 may apply transmit pulses for the transducer array TA to transmit an ultrasound signal to a target portion inside the object. The transmitter 100 may include a transmit beamformer 110 and a pulser 120.

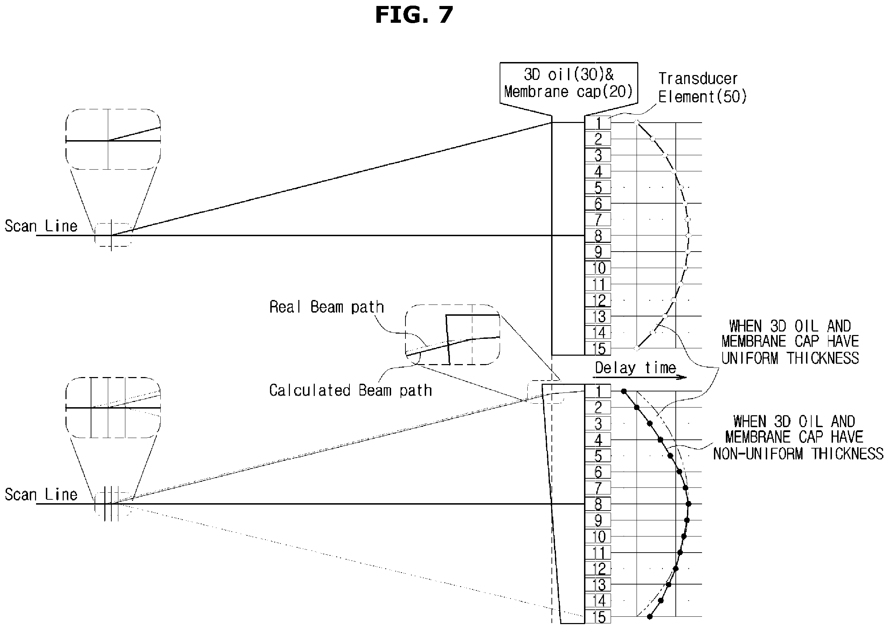

The transmit beamformer 110 forms a transmit signal pattern according to a control signal of the controller 500 of the main body M and outputs the transmit signal pattern to the pulser 120. The transmit beamformer 110 forms the transmit signal pattern based on time delay values for the respective ultrasonic transducer elements 50 that constitute the ultrasonic transducer array TA, the time delay values being calculated by the controller 500, and sends the transmit signal pattern to the pulser 120. The transmit beamforming will be described in detail in connection with FIG. 7.

The receiver 200 may perform a certain process and receive beamforming on the echo ultrasound received from the transducer array TA. The receiver 200 may include a receive beamformer 210 and a receive-signal processor 220. An electric signal converted by the transducer array TA may be input to the receive-signal processor 220. The receive-signal processor 220 may amplify the electric signal converted from the echo ultrasound prior to performing a signal process or a time-delay process, and adjust the gain or compensate for attenuations depending on the depth.

Specifically, the receive-signal processor 220 may include a low noise amplifier (LNA) for reducing noise of the electric signal input from the ultrasonic transducer array TA, and a variable gain amplifier (VGA) for adjusting a gain value based on the input signal. The VGA may correspond to a time gain compensation (TGC) amplifier that compensates for a gain based on a distance to the focal point, without being limited thereto.

The receive beamformer 210 may perform beamforming on the electric signal received from the receive-signal processor 220. The receive beamformer 210 may make the signal intensified through superposition of the electric signals received from the receive-signal processor 220. The signal beamformed by the receive beamformer 210 is converted to a digital signal by the AD converter, which is sent to the image processor 530 of the main body M. With the AD converter equipped in the main body M, an analog signal beamformed by the receive beamformer 210 may be sent to the main body M and then converted to a digital signal in the main body M. Alternatively, the receive beamformer 210 may be a digital beamformer.

The digital beamformer may include a storage for storing sampled analog signals, a sampling cycle controller for controlling a sampling cycle, an amplifier for adjusting the magnitude of the sample, an anti-aliasing low pass filter for preventing aliasing prior to sampling, a bandpass filter for selectively passing a desired frequency band, an interpolation filter for increasing a sampling rate when beamforming is performed, and a high pass filter for filtering out a direct current (DC) component or signals of a low frequency band. The receive beamforming will also be described in detail in connection with FIG. 7.

FIG. 7 is a view for explaining beamforming of ultrasonic transducer element signals, according to an embodiment of the present disclosure.

Although a 2D array transducer is used in this embodiment, transmit beamforming and receive beamforming will be described by taking an example of a 1D array transducer for convenience of explanation.

To improve the resolution of an ultrasound image, especially, the lateral resolution, an ultrasound beam with narrow beamwidth may be formed by focusing ultrasound signals transmitted from the plurality of transducer elements 50 to a focal point on the scan line, which is called transmit beamforming.

The 1D transducer array is comprised of the plurality of transducer elements 50 arrayed in one dimension. To obtain 2D ultrasound cross sectional images, a plurality of scan lines are required and beamforming may be performed for the focal point, as described above, from the first scan line till the last scan line.

A 2D ultrasound cross sectional image on the xy plane may be obtained by transmitting ultrasound signals for all the scan lines and receiving the echo ultrasound signals bouncing back from the internal substances of the object.

To focus ultrasound beams on a point, the ultrasound signals transmitted from the plurality of transducer elements 50 have to simultaneously reach the focal point. However, since the distances from the respective transducer elements 50 to the focal point are different, appropriate time delays are applied to the ultrasound signals to be transmitted from the transducer elements 50 (also referred to as 'elements') such that the ultrasound signals may reach the focal point at the same time. For example, the nearest element from the focal point may be given the latest transmit signal and the farthest element from the focal point may be give the earliest transmit signal. The transmit signal as herein used refers to an electric signal to be converted to an ultrasound signal by the element 50.

Referring to FIG. 7, if the membrane cap 20 and the 3D oil 30 at the front end of the ultrasonic probe P each have uniform thickness, the controller 500 needs to consider an increase in distance due to the uniform thickness for each of the ultrasonic transducer elements 50 to calculate a time delay value to be applied to the ultrasound signal to be transmitted by the element 50. Accordingly, the transmit beamforming result calculated by the controller 500 may correspond to an actual transmit beamforming result. In other words, the ultrasound signals transmitted by the respective ultrasonic transducer elements 50 with the time delay values calculated by the controller 500 may reach the focal point simultaneously.

On the other hand, if the membrane cap 20 and the 3D oil 30 at the front end of the ultrasonic probe P each have non-uniform thickness, the transmit beamforming result calculated by the controller 500 on the assumption that the membrane cap 20 and the 3D oil 30 each have uniform thickness, and an actual transmit beamforming result are different. For example, if the membrane cap 20 has different thickness at each position thereon, ultrasound signals transmitted by the respective ultrasonic transducer elements 50 are separately refracted with different refractive indexes and transmitted at the respective positions on the membrane cap 20. Accordingly, the ultrasound signals transmitted by the ultrasonic transducer elements 50 may not reach the single focal point simultaneously, and as a result, the resolution of the ultrasound image deteriorates.

Taking this into account, the thickness deviations at different positions need to be compensated for in the case that the membrane cap 20 and the 3D oil 30 each have non-uniform thickness. For example, the controller 500 obtains thickness data of at least one of the membrane cap 20 and the 3D oil 30 to compensate for distortions of signals transmitted or received by the ultrasonic transducer 40.

The controller 500 makes ultrasound signals transmitted by the ultrasonic transducer elements 50 simultaneously reach the single focal point despite the thickness deviations of the membrane cap 20 or the 3D oil 30 by calculating and applying time delay values to the respective signals transmitted by the plurality of ultrasonic transducer elements 50 by reflecting the thickness deviations of the membrane cap 20 or the 3D oil 30.

FIG. 7 also shows that the time delay values calculated by the controller 500 are applied such that one of the elements 50 located in the center is the last to transmit the ultrasound signal while the elements located at the outer ends are the first to transmit the ultrasound signal in the case that the thickness of the membrane cap 20 and the 3D oil 30 is not uniform.

The controller 500 may obtain thickness data of the membrane cap 20 for the entire area of the membrane cap 20 in the early stage of operation of the ultrasonic imaging apparatus 1, and compensate for thickness deviations of the respective positions on the membrane cap 20 based on the thickness data of the membrane cap 20 corresponding to the wavelength of the ultrasonic transducer signal.

Since distortions of the ultrasound signal may be minimized if the thickness of the membrane cap 20 corresponds to the wavelength of the ultrasound signal transmitted by the ultrasonic transducer 40, it is desirable to take the thickness data of the membrane cap 20 corresponding to the wavelength of the ultrasonic transducer signal as a reference.

Similar to the transmit beamforming, receive beamforming applies a time delay to a received signal. When ultrasound signals in phase reach the focal point by performing transmit beamforming, echo ultrasound signals are produced at the focal point and return to the transducer array TA. Similar to when the ultrasound signals are to be transmitted to the focal point, distances from the respective transducer elements 50 to the focal point are different when the echo ultrasound is received from the focal point, so the echo ultrasound signals arrive at the respective transducer elements 50 at different points in time. Specifically, the echo ultrasound signal arrives first at an element nearest to the focal point and arrives last at an element farthest from the focal point. Accordingly, time delays are applied to the echo ultrasound signals that reach the respective transducer elements 50 and the resultant echo ultrasound signals are combined at a time, thereby improving the signal to noise ratio.

The controller 500 may combine the ultrasound signals received by the ultrasonic transducer elements 50 at a time despite the thickness deviations of the membrane cap 20 or the 3D oil 30 by calculating and applying time delay values to the respective signals received by the plurality of ultrasonic transducer elements 50 by reflecting the thickness deviations of the membrane cap 20 or the 3D oil 30.

Furthermore, the controller 500 may obtain thickness data of the membrane cap 20 or the 3D oil 30 in real time and compensate for distortions of signals transmitted or received by the ultrasonic transducer 40. Since the thickness of the membrane cap 20 or the 3D oil 30 may vary by the pressure applied when the ultrasonic probe P contacts and scans the object, thickness data need to be obtained in real time to compensate for the thickness deviation.

Moreover, the controller 500 may determine the magnitude of the echo signal reflecting off on the border between the membrane cap 20 and the 3D oil 30 for the plurality of ultrasonic transducer elements 50, and regulate pulse voltages to be applied to the respective ultrasonic transducer elements 50 in order to level out the magnitude of the ultrasound signals transmitted by the plurality of transducer elements 50. The ultrasound signal reflects on the membrane cap 20, in which case energy loss or energy attenuation occurs as much as the reflected ultrasound energy, thereby reducing the ultrasound energy transmission efficiency. Accordingly, there is a need to compensate for the ultrasound energy loss by regulating the pulse voltages to be applied to the ultrasonic transducer elements 50.

Especially when the thickness of the membrane cap 20 or the 3D oil 30 is not uniform, the reflected echo ultrasound signals are different in magnitude at the respective positions on the membrane cap 20, so the pulse voltages to be applied to the respective ultrasonic transducer elements 50 are regulated to make the ultrasound signals transmitted by the plurality of ultrasonic transducer elements 50 have the uniform magnitude. Specifically, the controller 500 may regulate the pulse voltages to be applied to the respective ultrasonic transducer elements 50 to compensate for the difference in magnitude between the ultrasound signals transmitted by the respective ultrasonic transducer elements 50 and the received echo signals.

Furthermore, the controller 500 may obtain not only the thickness data of the membrane cap 20 but also the curvature data, and compensate for distortions of signals transmitted or received by the ultrasonic transducer 40 by reflecting the curvature data. Since the membrane cap 20 is formed to have a convexly curved plane, reflection or refraction of the ultrasound signal or the echo ultrasound signal may be different depending on the curvature of the membrane cap 20. In other words, an amount of ultrasound energy loss or attenuation may vary by the curvature of the membrane cap 20, so a compensation process on this is required. The controller 500 may calculate time delay values to be applied to signals transmitted or received by the respective ultrasonic transducer elements 50 by reflecting the curvature data of the membrane cap 20 and regulate pulse voltages to be applied to the respective ultrasonic transducer elements 50.

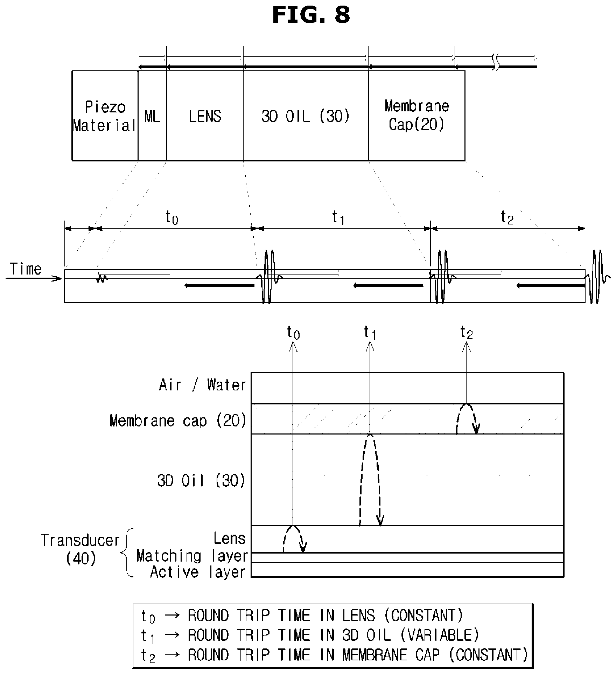

FIG. 8 is a view for explaining a method for obtaining thickness data of a membrane cap and 3D oil, according to an embodiment of the present disclosure.

Referring to FIG. 8, the controller 500 may obtain thickness data of the 3D oil 30 based on time t1 taken for an ultrasound signal from the ultrasonic transducer 40 to reflect off from the border between the membrane cap 20 and the 3D oil 30 and reach the surface of the ultrasonic transducer 40 and velocity of the ultrasound in the 3D oil 30.

Furthermore, the controller 500 may obtain thickness data of the membrane cap 20, based on time t2 taken for an ultrasound signal from the ultrasonic transducer 40 to reflect off from the outer surface of the membrane cap 20 and reach the border between the membrane cap 20 and the 3D oil 30 from when the ultrasound signal penetrates the border between the membrane cap 20 and the 3D oil 30, and velocity of the ultrasound in the membrane cap 20.

The point in time at which the ultrasound signal penetrates the border between the membrane cap 20 and the 3D oil 30 may be determined using a change in waveform of the ultrasound signal. Since the ultrasound signal transmitted by the ultrasonic transducer 40 reflects and transmits on the border between the membrane cap 20 and the 3D oil 30 by the nature of the medium, the waveform of the ultrasound signal reflecting off on the border may be detected to determine a point in time at which the ultrasound signal passes the border between the membrane cap 20 and the 3D oil 30.

In this way, the controller 500 may obtain thickness data of the membrane cap 20 or the 3D oil 30 in real time and compensate for distortions of the signal transmitted or received by the ultrasonic transducer 40.

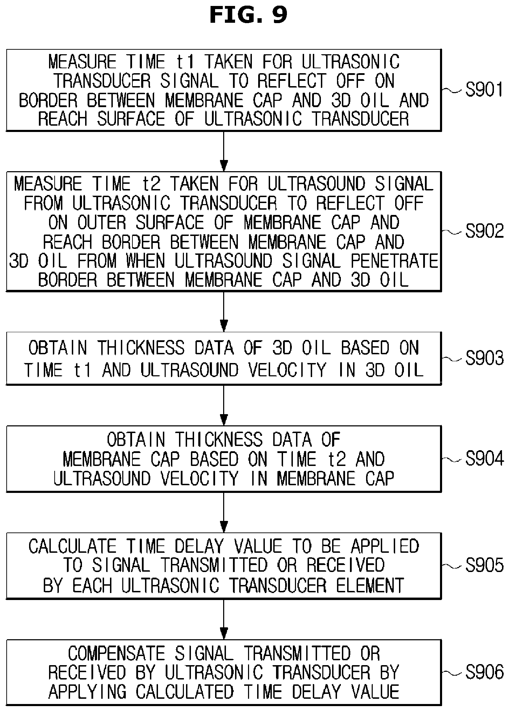

FIG. 9 is a flowchart illustrating a control method of an ultrasonic imaging apparatus, according to an embodiment of the present disclosure.

Referring to FIG. 9, the controller 500 of the ultrasonic imaging apparatus 1 measures time t1 taken for an ultrasound signal from the ultrasonic transducer 40 to reflect off from the border between the membrane cap 20 and the 3D oil 30 and reach the surface of the ultrasonic transducer 40 in S901, and measures time t2 taken for an ultrasound signal from the ultrasonic transducer 40 to reflect off from the outer surface of the membrane cap 20 and reach the border between the membrane cap 20 and the 3D oil 30 from when the ultrasound signal penetrates the border between the membrane cap 20 and the 3D oil 30, in S902.

The controller 500 calculates thickness data of the 3D oil 30 based on the time t1 and velocity of the ultrasound in the 3D oil 30, in S903, and calculates thickness data of the membrane cap 20 based on the time t2 and velocity of the ultrasound in the membrane cap 20, in S904.

The controller 500 calculates time delay values to be applied to signals transmitted or received by the respective ultrasonic transducer elements 50 based on the thickness data calculated for the membrane cap 20 and the 3D oil 30. The controller 500 compensates for distortions of signals transmitted or received by the ultrasonic transducer 40 by applying the calculated time delay values, in S906.

According to an embodiment of the present disclosure, an ultrasonic imaging apparatus and control method thereof is capable of compensating for distortions of ultrasonic transducer signals by measuring thicknesses of a membrane cap and 3D oil covering the ultrasonic transducer in real time without additional hardware, thereby saving manufacturing costs.

According to an embodiment of the present disclosure, an ultrasonic imaging apparatus and control method thereof is capable of improving the resolution of ultrasound images by compensating for thickness deviations of positions on a membrane cap and performing a compensation process on curvature changes in the membrane cap and thickness changes of 3D oil.

According to an embodiment of the present disclosure, an ultrasonic imaging apparatus and control method thereof is capable of securing uniformity of signal sensitivity of ultrasonic transducer elements by performing compensation for non-uniformity in thickness of a membrane cap or 3D oil.

Meanwhile, the embodiments of the present disclosure may be implemented in the form of recording media for storing instructions to be carried out by a computer. The instructions may be stored in the form of program codes, and when executed by a processor, may generate program modules to perform operation in the embodiments of the present disclosure. The recording media may correspond to computer-readable recording media.

The computer-readable recording medium includes any type of recording medium having data stored thereon that may be thereafter read by a computer. For example, it may be a ROM, a RAM, a magnetic tape, a magnetic disk, a flash memory, an optical data storage device, etc.

The embodiments of the present disclosure have thus far been described with reference to accompanying drawings. It will be obvious to people of ordinary skill in the art that the present disclosure may be practiced in other forms than the embodiments as described above without changing the technical idea or essential features of the present disclosure. The above embodiments are only by way of example, and should not be interpreted in a limited sense.

Thus, it will be apparent to those ordinary skilled in the art that the true scope of technical protection is only defined by the following claims.

## Claims

1. An ultrasonic imaging apparatus (1) comprising:
an ultrasonic probe (P) configured to have an ultrasonic transducer (40) with a plurality of elements (50), a membrane cap (20) provided in a form that covers the ultrasonic transducer, and three-dimensional (3D) oil (30) provided between the ultrasonic transducer (40) and the membrane cap (20);
a controller (500) configured to obtain at least one of thickness data of the membrane cap (20) or thickness data of the 3D oil (30), and calculate time delay values to be applied to the signals transmitted or received by the elements (50) of the ultrasonic transducer (40) to compensate for a distortion of a signal transmitted or received by the elements caused by at least one of a thickness deviation of positions on the membrane cap (20) or a thickness deviation of the 3D oil (30); and,
a display (550) configured to display an ultrasonic image generated based on said signals compensated by the controller.

2. The ultrasonic imaging apparatus (1) of claim 1, wherein the controller (500) is configured to obtain thickness data of the 3D oil (30) based on time taken for an ultrasound signal from the ultrasonic transducer (40) to reflect off on a border between the membrane cap (20) and the 3D oil (30) and reach a surface of the ultrasonic transducer (40) and ultrasound velocity in the 3D oil (30).

3. The ultrasonic imaging apparatus of claim 1, wherein the controller (500) is configured to obtain thickness data of the membrane cap (20) based on time for an ultrasound signal from the ultrasonic transducer (40) to reflect off on an outer surface of the membrane cap (20) and reach a border between the membrane cap (20) and the 3D oil (30) from when the ultrasound signal penetrates the border between the membrane cap (20) and the 3D oil (30) and ultrasound velocity in the membrane cap (20).

4. The ultrasonic imaging apparatus (1) of claim 1, wherein the controller (500) is configured to obtain thickness data of the membrane cap (20) for the entire area of the membrane cap (20) in the early stage of operation of the ultrasonic imaging apparatus (1), and calculate the time delay values to compensate for the thickness deviation of positions on the membrane cap (20) based on the thickness data of the membrane cap (20) corresponding to a wavelength of the signals of the ultrasonic transducer.

5. The ultrasonic imaging apparatus (1) of claim 1, wherein the controller (500) is configured to obtain thickness data of the membrane cap (20) or the 3D oil (30) in real time to compensate for a distortion of a signal transmitted or received by the ultrasonic transducer (40).

6. The ultrasonic imaging apparatus (1) of claim 1, wherein the controller (500) is configured to determine the magnitude of an echo signal reflecting off on a border between the membrane cap (20) and the 3D oil (30) for each of the plurality of ultrasonic transducer elements (50), and regulate a pulse voltage to be applied to each of the ultrasonic transducer elements (50) to make the magnitude of the ultrasound signals transmitted by the plurality of ultrasonic transducer elements (50) uniform.

7. The ultrasonic imaging apparatus (1) of claim 6, wherein the controller (500) is configured to regulate pulse voltages to be applied to the respective ultrasonic transducer elements (50) to compensate for differences in magnitude between ultrasound signals transmitted and echo signals received by the respective ultrasonic transducer elements (50).

8. A control method of an ultrasonic imaging apparatus (1) including an ultrasonic probe (P) configured to have an ultrasonic transducer (40) with a plurality of elements (50), a membrane cap (20) provided in a form that covers the ultrasonic transducer (40), and three-dimensional (3D) oil (30) provided between the ultrasonic transducer (40) and the membrane cap (20), the control method comprising:
obtaining at least one of thickness data of the membrane cap (20) or thickness data of the 3D oil (30);
calculating time delay values to be applied to the signals transmitted or received by the elements (50) of the ultrasonic transducer (40) to compensate for a distortion of a signal transmitted or received by the elements caused by at least one of a thickness deviation of positions on the membrane cap (20) or a thickness deviation of the 3D oil (30); and
displaying an ultrasonic image generated based on the compensated signals;

9. The control method of claim 8, wherein obtaining thickness data comprises:
obtaining thickness data of the 3D oil (30) based on time taken for an ultrasound signal from the ultrasonic transducer (40) to reflect off on a border between the membrane cap (20) and the 3D oil (30) and reach a surface of the ultrasonic transducer (40) and ultrasound velocity in the 3D oil (30).

10. The control method of claim 8, wherein obtaining thickness data comprises:
obtaining thickness data of the membrane cap (20) based on time for an ultrasound signal from the ultrasonic transducer (40) to reflect off on an outer surface of the membrane cap (20) and reach a border between the membrane cap (20) and the 3D oil (30) from when the ultrasound signal penetrates the border between the membrane cap (20) and the 3D oil (30) and ultrasound velocity in the membrane cap (20).

11. The control method of claim 8, wherein obtaining thickness data comprises:
obtaining thickness data of the membrane cap (20) for the entire area of the membrane cap (20) in the early stage of operation of the ultrasonic imaging apparatus (1), and
wherein calculating time delay values comprises
calculating time delay values to compensate for the thickness deviation of positions on the membrane cap (20) based on the thickness data of the membrane cap (20) corresponding to a wavelength of the signals of the ultrasonic transducer.

12. The control method of claim 8, wherein obtaining thickness data comprises:
obtaining thickness data of the membrane cap (20) or the 3D oil (30) in real time.

13. The control method of claim 8, further comprising:
determining the magnitude of an echo signal reflecting off on a border between the membrane cap (20) and the 3D oil (30) for each of the plurality of ultrasonic transducer elements (50), and
regulating a pulse voltage to be applied to each of the ultrasonic transducer elements (50) to make the magnitude of the ultrasound signals transmitted by the plurality of ultrasonic transducer elements (50) uniform.

## Patentansprüche

1. Ultraschallbildgebungsvorrichtung (1), die Folgendes umfasst:
eine Ultraschallsonde (P), die so ausgelegt ist, dass sie Folgendes aufweist: einen Ultraschallwandler (40) mit einer Vielzahl von Elementen (50), eine Membrankappe (20), die in einer Form vorgesehen ist, die den Ultraschallwandler bedeckt, und dreidimensionales Öl (3D-Öl) (30), das zwischen dem Ultraschallwandler (40) und der Membrankappe (20) vorgesehen ist;
eine Steuerung (500), die so ausgelegt ist, dass sie Dickendaten der Membrankappe (20) und/oder Dickendaten des 3D-Öls (30) erhält und Zeitverzögerungswerte berechnet, die auf die von den Elementen (50) des Ultraschallwandlers (40) gesendeten oder empfangenen Signale anzuwenden sind, um eine durch eine Dickenabweichung von Positionen auf der Membrankappe (20) und/oder eine Dickenabweichung des 3D-Öls (30) verursachte Verzerrung eines von den Elementen gesendeten oder empfangenen Signals zu kompensieren; und
eine Anzeige (550), die so ausgelegt ist, dass sie ein Ultraschallbild anzeigt, das basierend auf den durch die Steuerung kompensierten Signalen erzeugt wird.

2. Ultraschallbildgebungsvorrichtung (1) nach Anspruch 1, wobei die Steuerung (500) dazu ausgelegt ist, Dickendaten des 3D-Öls (30) zu erhalten basierend auf der Zeit, die ein Ultraschallsignal von dem Ultraschallwandler (40) benötigt, um an einer Grenze zwischen der Membrankappe (20) und dem 3D-Öl (30) abzureflektieren und eine Oberfläche des Ultraschallwandlers (40) zu erreichen, und auf der Ultraschallgeschwindigkeit in dem 3D-Öl (30).

3. Ultraschallbildgebungsvorrichtung nach Anspruch 1, wobei die Steuerung (500) dazu ausgelegt ist, Dickendaten der Membrankappe (20) zu erhalten basierend auf der Zeit, die ein Ultraschallsignal von dem Ultraschallwandler (40) benötigt, um an einer Außenfläche der Membrankappe (20) abzureflektieren und eine Grenze zwischen der Membrankappe (20) und dem 3D-Öl (30) zu erreichen, von dem Moment, an dem das Ultraschallsignal die Grenze zwischen der Membrankappe (20) und dem 3D-Öl (30) durchdringt, und auf der Ultraschallgeschwindigkeit in der Membrankappe (20).

4. Ultraschallbildgebungsvorrichtung (1) nach Anspruch 1, wobei die Steuerung (500) dazu ausgelegt ist, Dickendaten der Membrankappe (20) für den gesamten Bereich der Membrankappe (20) in der frühen Betriebsphase der Ultraschallbildgebungsvorrichtung (1) zu erhalten und die Zeitverzögerungswerte zum Kompensieren der Dickenabweichung von Positionen auf der Membrankappe (20) basierend auf den Dickendaten der Membrankappe (20) entsprechend einer Wellenlänge der Signale des Ultraschallwandlers zu berechnen.

5. Ultraschallbildgebungsvorrichtung (1) nach Anspruch 1, wobei die Steuerung (500) dazu ausgelegt ist, Dickendaten der Membrankappe (20) oder des 3D-Öls (30) in Echtzeit zu erhalten, um eine Verzerrung eines von dem Ultraschallwandler (40) gesendeten oder empfangenen Signals zu kompensieren.

6. Ultraschallbildgebungsvorrichtung (1) nach Anspruch 1, wobei die Steuerung (500) dazu ausgelegt ist, die Größe eines Echosignals, das an einer Grenze zwischen der Membrankappe (20) und dem 3D-Öl (30) abreflektiert wird, für jedes aus der Vielzahl von Ultraschallwandlerelementen (50) zu bestimmen, eine an jedes der Ultraschallwandlerelemente (50) anzulegende Impulsspannung zu regeln, um die Größe der von der Vielzahl von Ultraschallwandlerelementen (50) gesendeten Ultraschallsignale gleichförmig zu machen.

7. Ultraschallbildgebungsvorrichtung (1) nach Anspruch 6, wobei die Steuerung (500) so ausgelegt ist, dass sie an die jeweiligen Ultraschallwandlerelemente (50) anzulegende Impulsspannungen regelt, um Größenunterschiede zwischen von den jeweiligen Ultraschallwandlerelementen (50) gesendeten Ultraschallsignalen und empfangenen Echosignalen zu kompensieren.

8. Steuerungsverfahren für eine Ultraschallbildgebungsvorrichtung (1) mit einer Ultraschallsonde (P), die so ausgelegt ist, dass sie Folgendes aufweist: einen Ultraschallwandler (40) mit einer Vielzahl von Elementen (50), eine Membrankappe (20), die in einer Form vorgesehen ist, die den Ultraschallwandler (40) bedeckt, und dreidimensionales Öl (3D-Öl) (30), das zwischen dem Ultraschallwandler (40) und der Membrankappe (20) vorgesehen ist, wobei das Steuerungsverfahren Folgendes umfasst:
Erhalten von Dickendaten der Membrankappe (20) und/oder Dickendaten des 3D-Öls (30);
Berechnen von Zeitverzögerungswerten, die auf die von den Elementen (50) des Ultraschallwandlers (40) gesendeten oder empfangenen Signale anzuwenden sind, um eine durch eine Dickenabweichung von Positionen auf der Membrankappe (20) und/oder eine Dickenabweichung des 3D-Öls (30) verursachte Verzerrung eines von den Elementen gesendeten oder empfangenen Signals zu kompensieren; und
Anzeigen eines basierend auf den kompensierten Signalen erzeugten Ultraschallbildes.

9. Steuerungsverfahren nach Anspruch 8, wobei das Erhalten der Dickendaten Folgendes umfasst: Erhalten von Dickendaten des 3D-Öls (30) basierend auf der Zeit, die ein Ultraschallsignal von dem Ultraschallwandler (40) benötigt, um an einer Grenze zwischen der Membrankappe (20) und dem 3D-Öl (30) abzureflektieren und eine Oberfläche des Ultraschallwandlers (40) zu erreichen, und auf der Ultraschallgeschwindigkeit in dem 3D-Öl (30).

10. Steuerungsverfahren nach Anspruch 8, wobei das Erhalten der Dickendaten Folgendes umfasst: Erhalten von Dickendaten der Membrankappe (20) basierend auf der Zeit, die ein Ultraschallsignal von dem Ultraschallwandler (40) benötigt, um an einer Außenfläche der Membrankappe (20) abzureflektieren und eine Grenze zwischen der Membrankappe (20) und dem 3D-Öl (30) zu erreichen, von dem Moment, an dem das Ultraschallsignal die Grenze zwischen der Membrankappe (20) und dem 3D-Öl (30) durchdringt, und auf der Ultraschallgeschwindigkeit in der Membrankappe (20).

11. Steuerungsverfahren nach Anspruch 8, wobei das Erhalten der Dickendaten Folgendes umfasst:
Erhalten von Dickendaten der Membrankappe (20) für den gesamten Bereich der Membrankappe (20) in der frühen Betriebsphase der Ultraschallbildgebungsvorrichtung (1), und
wobei das Berechnen der Zeitverzögerungswerte Folgendes umfasst:
Berechnen der Zeitverzögerungswerte, um die Dickenabweichung von Positionen auf der Membrankappe (20) basierend auf den Dickendaten der Membrankappe (20) entsprechend einer Wellenlänge der Signale des Ultraschallwandlers zu kompensieren.

12. Steuerungsverfahren nach Anspruch 8, wobei das Erhalten der Dickendaten Folgendes umfasst: Erhalten von Dickendaten der Membrankappe (20) oder des 3D-Öls (30) in Echtzeit.

13. Steuerungsverfahren nach Anspruch 8, das ferner Folgendes umfasst:
Bestimmen der Größe eines Echosignals, das an einer Grenze zwischen der Membrankappe (20) und dem 3D-Öl (30) für jedes aus der Vielzahl von Ultraschallwandlerelementen (50) reflektiert wird, und
Regeln einer an jedes der Ultraschallwandlerelemente (50) anzulegenden Impulsspannung, um die Größe der von der Vielzahl von Ultraschallwandlerelementen (50) gesendeten Ultraschallsignale gleichförmig zu machen.

## Revendications

1. Appareil échographique (1) comprenant :
une sonde échographique (P) conçue pour présenter un transducteur ultrasonore (40) doté d'une pluralité d'éléments (50), un capuchon membranaire (20) dont la forme permet de recouvrir le transducteur ultrasonore, et de l'huile tridimensionnelle (3D) (30) prévue entre le transducteur ultrasonore (40) et le capuchon membranaire (20) ;
un organe de commande (500) conçu pour obtenir des données d'épaisseur concernant le capuchon membranaire (20) et/ou des données d'épaisseur concernant l'huile 3D (30), et pour calculer des valeurs de temporisation destinées à être appliquées aux signaux émis ou reçus par les éléments (50) du transducteur ultrasonore (40) pour compenser une distorsion d'un signal, émis ou reçu par les éléments, causée par un écart d'épaisseur à certains emplacements du capuchon membranaire (20) et/ou un écart d'épaisseur de l'huile 3D (30) ; et
un écran (550) conçu pour afficher une image échographique générée compte tenu desdits signaux compensés par l'organe de commande.

2. Appareil échographique (1) selon la revendication 1, dans lequel l'organe de commande (500) est conçu pour obtenir des données d'épaisseur concernant l'huile 3D (30) compte tenu du temps pris par un signal ultrasonore émis par le transducteur ultrasonore (40) pour être réfléchi par l'interface entre le capuchon membranaire (20) et l'huile 3D (30) et pour atteindre la surface du transducteur ultrasonore (40) et compte tenu de la vitesse des ultrasons dans l'huile 3D (30).

3. Appareil échographique selon la revendication 1, dans lequel l'organe de commande (500) est conçu pour obtenir des données d'épaisseur concernant le capuchon membranaire (20) compte tenu du temps nécessaire à un signal ultrasonore émis par le transducteur ultrasonore (40) pour être réfléchi par la surface extérieure du capuchon membranaire (20) et pour atteindre l'interface entre le capuchon membranaire (20) et l'huile 3D (30) à partir du moment auquel le signal ultrasonore pénètre dans l'interface entre le capuchon membranaire (20) et l'huile 3D (30) et compte tenu de la vitesse des ultrasons dans le capuchon membranaire (20).

4. Appareil échographique (1) selon la revendication 1, dans lequel l'organe de commande (500) est conçu pour obtenir des données d'épaisseur concernant le capuchon membranaire (20) pour la totalité de la zone formée par le capuchon membranaire (20) au début de la phase de fonctionnement de l'appareil échographique (1), et pour calculer les valeurs de temporisation pour compenser l'écart d'épaisseur aux emplacements du capuchon membranaire (20) compte tenu des données d'épaisseur concernant le capuchon membranaire (20) en correspondance avec la longueur d'onde des signaux du transducteur ultrasonore.

5. Appareil échographique (1) selon la revendication 1, dans lequel l'organe de commande (500) est conçu pour obtenir des données d'épaisseur concernant le capuchon membranaire (20) ou l'huile 3D (30) en temps réel pour compenser une distorsion d'un signal émis ou reçu par le transducteur ultrasonore (40).

6. Appareil échographique (1) selon la revendication 1, dans lequel l'organe de commande (500) est conçu pour déterminer la grandeur d'un signal d'écho réfléchi par l'interface entre le capuchon membranaire (20) et l'huile 3D (30) pour chacun des éléments de la pluralité d'éléments (50) du transducteur ultrasonore, réguler une tension d'impulsion destinée à être appliquée à chacun des éléments (50) du transducteur ultrasonore afin de rendre uniforme la grandeur des signaux ultrasonores émis par la pluralité d'éléments (50) du transducteur ultrasonore.

7. Appareil échographique (1) selon la revendication 6, dans lequel l'organe de commande (500) est conçu pour réguler les tensions d'impulsion destinées à être appliquées aux divers éléments (50) du transducteur ultrasonore pour compenser les différences de grandeur entre les signaux ultrasonores émis et les signaux d'écho reçus par les divers éléments (50) du transducteur ultrasonore.

8. Procédé de commande d'un appareil échographique (1) comportant une sonde échographique (P) conçue pour présenter un transducteur ultrasonore (40) doté d'une pluralité d'éléments (50), un capuchon membranaire (20) dont la forme permet de recouvrir le transducteur ultrasonore (40), et de l'huile tridimensionnelle (3D) (30) prévue entre le transducteur ultrasonore (40) et le capuchon membranaire (20), le procédé de commande comprenant :
l'obtention de données d'épaisseur concernant le capuchon membranaire (20) et/ou de données d'épaisseur concernant l'huile 3D (30) ;
le calcul de valeurs de temporisation destinées à être appliquées aux signaux émis ou reçus par les éléments (50) du transducteur ultrasonore (40) pour compenser une distorsion d'un signal, émis ou reçu par les éléments, causée par un écart d'épaisseur à certains emplacements du capuchon membranaire (20) et/ou un écart d'épaisseur de l'huile 3D (30) ; et
l'affichage d'une image échographique générée compte tenu des signaux compensés.

9. Procédé de commande selon la revendication 8, dans lequel l'obtention des données d'épaisseur comprend :
l'obtention de données d'épaisseur concernant l'huile 3D (30) compte tenu du temps pris par un signal ultrasonore émis par le transducteur ultrasonore (40) pour être réfléchi par l'interface entre le capuchon membranaire (20) et l'huile 3D (30) et pour atteindre la surface du transducteur ultrasonore (40) et compte tenu de la vitesse des ultrasons dans l'huile 3D (30).

10. Procédé de commande selon la revendication 8, dans lequel l'obtention des données d'épaisseur comprend :
l'obtention de données d'épaisseur concernant le capuchon membranaire (20) compte tenu du temps nécessaire à un signal ultrasonore émis par le transducteur ultrasonore (40) pour être réfléchi par la surface extérieure du capuchon membranaire (20) et pour atteindre l'interface entre le capuchon membranaire (20) et l'huile 3D (30) à partir du moment auquel le signal ultrasonore pénètre dans l'interface entre le capuchon membranaire (20) et l'huile 3D (30) et compte tenu de la vitesse des ultrasons dans le capuchon membranaire (20).

11. Procédé de commande selon la revendication 8, dans lequel l'obtention des données d'épaisseur comprend :
l'obtention de données d'épaisseur concernant le capuchon membranaire (20) pour la totalité de la zone formée par le capuchon membranaire (20) au début de la phase de fonctionnement de l'appareil échographique (1), et
dans lequel le calcul des valeurs de temporisation comprend :
le calcul des valeurs de temporisation pour compenser l'écart d'épaisseur en des emplacements du capuchon membranaire (20), compte tenu des données d'épaisseur concernant le capuchon membranaire (20), en correspondance avec la longueur d'onde des signaux du transducteur ultrasonore.

12. Procédé de commande selon la revendication 8, dans lequel l'obtention des données d'épaisseur comprend :
l'obtention de données d'épaisseur concernant le capuchon membranaire (20) ou l'huile 3D (30) en temps réel.

13. Procédé de commande selon la revendication 8, comprenant en outre :
la détermination de la grandeur d'un signal d'écho réfléchi par l'interface entre le capuchon membranaire (20) et l'huile 3D (30) pour chacun des éléments de la pluralité d'éléments (50) du transducteur ultrasonore, et
la régulation de la tension d'impulsion destinée à être appliquée à chacun des éléments (50) du transducteur ultrasonore afin de rendre uniforme la grandeur des signaux ultrasonores émis par la pluralité d'éléments (50) du transducteur ultrasonore.
